# EUROPEAN PATENT APPLICATION

(11) **EP 4 516 307 A1**
(43) Date of publication of application: **05.03.2025**
(21) Application number: 23815232.6
(22) Date of filing: 31.05.2023
(51) Int. Cl.: A61K 36/9068, A61K 36/884, A61K 36/752, A61K 36/725, A61K 36/71, A61K 36/54, A61K 36/486, A61K 36/481, A61K 36/284, A61K 36/28, A61K 36/25, A61K 36/16, A61K 36/076, A61P 13/12, A61P 9/04, A61P 9/00

(54) **TRADITIONAL CHINESE MEDICINE COMPOSITION FOR TREATING CARDIOVASCULAR AND KIDNEY DISEASES AND METHOD FOR PREPARING SAME**

(30) Priority: 01.06.2022 CN 202210615447
(71) Applicant: Beijing Capitalbio Pharma Co., Ltd., Beijing 102206 (CN)
(72) Inventor: CHENG, Jing, Beijing 102206 (CN); QIAO, Liansheng, Beijing 102206 (CN); XIE, Lan, Beijing 102206 (CN); GUO, Hongyan, Beijing 102206 (CN); ZHANG, Zhi, Beijing 102206 (CN)
(74) Representative: ABG Intellectual Property Law, S.L.
(86) International application number: PCT/CN2023/097312
(87) International publication number: WO 2023/232054

(57) **Abstract**

Provided are a traditional Chinese medicine composition for treating cardiorenal syndrome and cardiac failure and a method for preparing same. The traditional Chinese medicine composition is prepared from the following raw materials: 3 to 20 parts of *Cinnamomi ramulus,* 3 to 25 parts of *Atractylodis macrocephalae rhizoma,* 3 to 25 parts of *Jujubae fructus,* 6 to 45 parts of *Astragali radix,* 2 to 20 parts of *Panacis majoris rhizoma, 3* to 30 parts of *Paeoniae rubra radix,* 6 to 25 parts of *Spatholobi caulis,* 6 to 25 parts of *Ginkgo folium,* 6 to 35 parts of *Poria,* 3 to 20 parts of *Zingiberis rhizoma recens,* 2 to 25 parts of *Citri fructus,* 2 to 15 parts of *Nigellae semen,* 2 to 20 parts of *Inulae flos,* and 3 to 25 parts of *Alismatis rhizoma.* The traditional Chinese medicine composition has a regulatory effect on symptoms such as myocardial ischemia, myocardial hypertrophy, myocardial fibrosis, and renal fibrosis caused by cardiac failure and cardiorenal syndrome.

## Description

This application claims the priority of Chinese Patent Application No. 202210615447.4, filed with the China National Intellectual Property Administration on June 01, 2022, and titled with "TRADITIONAL CHINESE MEDICINE COMPOSITION FOR TREATING CARDIOVASCULAR AND KIDNEY DISEASES AND METHOD FOR PREPARING SAME", the disclosure of which is hereby incorporated by its reference in entirety.

### FIELD

The present disclosure relates to the technical field of traditional Chinese medicine, and in particular to a traditional Chinese medicine composition for treating cardiovascular and kidney diseases and a preparation method thereof.

### BACKGROUND

Heart failure is a vicious cycle that impaired cardiac or vascular function causes sympathetic activation, which leads to continued activation of the renin-angiotensin-aldosterone system, resulting in continued impairment of cardiac function. Cardiorenal syndrome refers to a disease in which acute and chronic functional abnormalities occur of the heart and/or kidneys lead to further acute and chronic functional abnormalities of them. The manifestation on the heart is a continuous decrease in ejection fraction, a continuous decrease in blood output, and a slowdown in blood flow. On this basis, the compensatory function of the heart will be activated, leading to myocardial ischemia, myocardial hypertrophy and myocardial fibrosis, leading to the continuous development of heart failure. The manifestation on the renal is a renal damage and a development of a pathological state of renal fibrosis, which leads to increased reabsorption of sodium and water, decreased urine output, and oedema. From the perspective of traditional Chinese medicine, the pathogenesis of heart failure and cardiorenal syndrome is qi deficiency, blood stasis, and obstruction of communication between the heart and kidney, which will lead to deficiency of heart and yang, phlegm-fluid retention, and water accumulation over time.

Therefore, from the perspective of Chinese medicine theory, synergistically regulating heart and kidney functions, warming yang and tonifying vital energy, promoting blood circulation and removing blood stasis, removing phlegm and promoting urination are the core treatments for heart failure and cardiorenal syndrome. The treatment of heart failure and cardiorenal syndrome with traditional Chinese medicine has unique clinical advantages, but there is still no special Chinese medicine composition that can effectively treat heart failure and cardiorenal syndrome, and the efficacy of Western medicine for the treatment of heart failure is poor, and the efficacy of improving symptoms is not strong.

### SUMMARY

In view of this, the present disclosure provides a traditional Chinese medicine composition for treating cardiovascular and kidney diseases and a preparation method thereof. The composition provided in the present disclosure can significantly reduce the gene expression of natriuretic peptide B-type (NPPB), a gold index of heart failure induced by hypoxia/reoxygenation, reduce the expression of genes related to TGFβ-induced myocardial fibrosis and renal fibrosis, and significantly increase the blood flow rate and cardiac output in the zebrafish heart failure model. It is indicated that the composition has a regulatory effect on the pathological developments such as myocardial ischemia, myocardial hypertrophy, myocardial fibrosis, renal fibrosis caused by heart failure and cardiorenal syndrome. It can effectively improve the cardiac renal function, inhibit the progression of heart failure and inhibit cardiorenal fibrosis, thereby achieving the therapeutic effect of treating cardiorenal syndrome and heart failure.

In order to achieve the above objects, the present disclosure provides the following technical solutions.

The present disclosure provides a traditional Chinese medicine composition, comprising the following components or processed products thereof in parts by weight: 3 to 20 parts of *Cinnamomi ramulus,* 3 to 25 parts of *Atractylodis macrocephalae rhizoma,* 3 to 25 parts of *Jujubae fructus,* 6 to 45 parts of *Astragali radix,* 2 to 20 parts of *Panacis majoris rhizoma,* 3 to 30 parts of *Paeoniae rubra radix,* 6 to 25 parts of *Spatholobi caulis,* 6 to 25 parts of *Ginkgo folium,* 6 to 35 parts of *Poria,* 3 to 20 parts of *Zingiberis rhizoma recens,* 2 to 25 parts of *Citri fructus,* 2 to 15 parts of *Nigellae semen,* 2 to 20 parts of *Inulae flos,* and 3 to 25 parts of *Alismatis rhizoma.*

In some embodiments of the present disclosure, *Cinnamomi ramulus* is dried young branchs of *Cinnamomum cassia* Presl (Lauraceae family), which can warm and unblock the meridians, warm yang, and benefit vital energy.

*Atractylodis macrocephalae rhizoma* is the dried rhizome of *Atractylodes macrocephala* Koidz (Asteraceae family), which can invigorate spleen and benefit vital energy, dry dampness and promote diuresis.

*Jujubae fructus* is the dried ripe fruit of *Ziziphus jujuba* Mill (Rhamnaceae family), which can tonify middle burner, benefit vital energy, nourish the blood, and calm the mind.

*Astragali radix* is the dried root of *Astragalus membranaceus* (Fisch.) Bge. var. mongholicus ( Bge.) Hsiao or *Astragalus membranaceus* (Fisch.) Bge. (Leguminosae family), which can tonify vital energy, upraise yang, promote urination, resolve oedema, engender body fluids, nourish blood, activate stagnation and obstruction.

*Panacis majoris rhizoma* is the dried rhizome of *Panax japonicus* C. A. Mey. var. major (Burk.) C. Y. Wu et K. M Feng or *Panax japonicus* C. A. Mey. var. bipinnatifidus (Seem.) C. Y. Wu et K. M. Feng (Araliaceae family), which can tonify lung, nourish yin, and eliminate stasis. It is Bai ethnic medicine that is useful in the treatment of dual deficiency of vital energy and yin, heart failure and cardiorenal syndromes.

*Paeoniae rubra radix* is the dried root of *Paeonia lactiflora* PalL or *Paeonia veitchii* Lynch (Ranunculaceae family), which can clear away heat, cool blood, dissipate stasis, and relieve pain.

*Spatholobi caulis* is the dried lianoid stem of *Spatholobus suberectus* Dunn (Leguminosae family), which can invigorate blood circulation, replenish blood, relax tendons and activate meridians.

*Ginkgo folium* is the dried leaf of *Ginkgo biloba* L. (Ginkgoaceae family), which can promote blood circulation, resolve stasis, dredge meridians, relieve pain, astringe lungs, relieve asthma, resolve turbidity and lower lipid.

*Poria* is the dried sclerotium of the fungus, *Poria cocos* (Schw.) Wolf (Polyporaceae family), which can promote urination, drain dampness, fortify spleen, and calm the heart.

*Zingiberis rhizoma recens* is the fresh rhizome of *Zingiber officinale* Rosc. (Zingiberaceae family), which can warm yang, resolve phlegm, and suppress cough.

*Citri fructus* is the dried ripe fruit of *Citrus medica* L. or *Citrus wilsonii* Tanaka (Rutaceae family), which can soothe middle burner, resolve phlegm, soothe liver and regulate vital energy.

*Nigellae semen* is the dried mature seed of *Nigella glandulifera Freyn* et Sint. (Ranunculaceae family), which is a Uygur ethnic medicine that can tonify kidney and promote diuresis.

*Inulae flos* is the dried capitulum of *Inula japonica* Thunb. or *Inula britannica* L*.* (Compositae family), which can make vital energy downward, resolve phlegm, and regulate water.

*Alismatis rhizoma* is the dried tuber of *Alisma orientale* (Sam.) Juzep. (Alismataceae family), which can promote urination, drain dampness, resolve turbidity, and lower lipid.

In some embodiments of the present disclosure, the above-mentioned traditional Chinese medicine composition comprises the following components or processed products thereof: 9 parts of *Cinnamomi ramulus,* 10 parts of *Atractylodis macrocephalae rhizoma,* 10 parts of *Jujubae fructus,* 20 parts of *Astragali radix,* 9 parts of *Panacis majoris rhizoma,* 12 parts of *Paeoniae rubra radix,* 10 parts of *Spatholobi caulis,* 10 parts of *Ginkgo folium,* 15 parts of *Poria,* 9 parts of *Zingiberis rhizoma recens,* 10 parts of *Citri fructus,* 6 parts of *Nigellae semen,* 9 parts of *Inulae flos,* and 10 parts of *Alismatis rhizoma.*

In some embodiments of the present disclosure, in the above-mentioned traditional Chinese medicine composition,
the *Panacis majoris rhizoma* is replaced by Ginseng radix et rhizoma, Ginseng radix et Rhizoma rubra, Codonopsis radix, Panacis japonici rhizoma, Panacis quinquefolii radix, and/ or Pseudostellariae radix, and/or
the *Atractylodis macrocephalae rhizoma* is stir-baked *Atractylodis macrocephalae rhizoma* or stir-baked *Atractylodis macrocephalae rhizoma* with bran, and/or
the *Astragali radix* is Radix astragali preparata, and/or
the *Paeoniae rubra radix* is replaced by Paeoniae radix alba, and/or
the *Zingiberis rhizoma recens* is dried *Zingiberis rhizoma recens* or pickled *Zingiberis rhizoma recens.*

The present disclosure further provides a method for preparing the traditional Chinese medicine composition, comprising extracting the components or processed products thereof of the traditional Chinese medicine composition in a formulated amount with water.

In some embodiments of the present disclosure, the method further comprises steps of concentrating and/or drying after the extracting with water.

In some embodiments of the present disclosure, the method further comprises crushing the components or processed products thereof of the traditional Chinese medicine composition in a formulated amount to obtain a fine powder, and then extracting with water, concentrating, and freeze-drying.

In some embodiments of the present disclosure, the extracting with water comprises decocting with water.

The present disclosure further provides use of the above-mentioned traditional Chinese medicine composition and/or the traditional Chinese medicine composition prepared by the above-mentioned method in the manufacture of a medicament for the regulation of cardiac and renal functions.

The present disclosure further provides use of the above-mentioned traditional Chinese medicine composition and/or the traditional Chinese medicine composition prepared by the above-mentioned method in the manufacture of a medicament for the prevention and/or treatment of heart failure, cardiorenal syndrome and/or coronary heart disease.

The present disclosure further provides use of the above-mentioned traditional Chinese medicine composition and/or the traditional Chinese medicine composition prepared by the above-mentioned method in the manufacture of a medicament for increasing the blood flow rate and/or cardiac output in a zebrafish model of heart failure induced by verapamil.

The present disclosure further provides use of the above-mentioned traditional Chinese medicine composition and/or the traditional Chinese medicine composition prepared by the above-mentioned method in the manufacture of a medicament for the inhibition of myocardial ischaemia, myocardial hypertrophy, myocardial fibrosis and/or renal fibrosis.

The present disclosure further provides use of the above-mentioned traditional Chinese medicine composition and/or the traditional Chinese medicine composition prepared by the above-mentioned method in the manufacture of a medicament for the improvement of a symptom of a patient with chronic heart failure and/or the reduction of the NT-proBNP expression.

In some embodiments of the present disclosure, in the above use, the symptom includes one or more of oedema, wheezing, tiredness and weakness and/or shortness of breath.

The present disclosure further provides use of the above-mentioned traditional Chinese medicine composition and/or the traditional Chinese medicine composition prepared by the above-mentioned method in the manufacture of a medicament for the inhibition of the expression of *NPPB* gene, *COL1A1* gene, *COL5A1* gene and/or *SERPINE1* gene.

The present disclosure further provides use of the above-mentioned traditional Chinese medicine composition in the clinical treatment of chronic heart failure, including improving a symptom of a patient and lowering the NT-proBNP level in the plasma.

The present disclosure further provides a medicament comprising the above-mentioned traditional Chinese medicine composition and/or the traditional Chinese medicine composition prepared by the above-mentioned method, and a pharmaceutically acceptable excipient.

The present disclosure further provides use of the above-mentioned traditional Chinese medicine composition, the traditional Chinese medicine composition prepared by the above-mentioned method, and/or the above-mentioned medicament in the treatment of a disease selected from the group consisting of:
(I) heart failure,
(II) cardiorenal syndrome,
(III) coronary heart disease,
(IV) myocardial ischaemia,
(V) myocardial hypertrophy,
(VI) myocardial fibrosis, and
(VII) renal fibrosis.

The present disclosure further provides a method for treating a disease, comprising administering the above-mentioned traditional Chinese medicine composition, the traditional Chinese medicine composition prepared by the above-mentioned method, and/or the above-mentioned medicament, wherein the disease is selected from the group consisting of:
(I) heart failure,
(II) cardiorenal syndrome,
(III) coronary heart disease,
(IV) myocardial ischaemia,
(V) myocardial hypertrophy,
(VI) myocardial fibrosis, and
(VII) renal fibrosis.

The present disclosure provides a traditional Chinese medicine composition, comprising the following components or processed products thereof in parts by weight: 3 to 20 parts of *Cinnamomi ramulus,* 3 to 25 parts of *Atractylodis macrocephalae rhizoma,* 3 to 25 parts of *Jujubae fructus,* 6 to 45 parts of *Astragali radix,* 2 to 20 parts of *Panacis majoris rhizoma,* 3 to 30 parts of *Paeoniae rubra radix,* 6 to 25 parts of *Spatholobi caulis,* 6 to 25 parts of *Ginkgo folium,* 6 to 35 parts of *Poria,* 3 to 20 parts of *Zingiberis rhizoma recens,* 2 to 25 parts of *Citri fructus,* 2 to 15 parts of *Nigellae semen,* 2 to 20 parts of *Inulae flos,* and 3 to 25 parts of *Alismatis rhizoma.*

In this traditional Chinese medicine composition, *Astragali radix* and *Nigellae semen* are used as sovereign medicines, in which *Astragali radix* can tonify vital energy, upraise yang, promote urination, and resolve oedema, and *Nigellae semen* can tonify kidney and promote diuresis. Therefore, the combination of *Astragali radix* and *Nigellae semen* can be used to treat heart failure and cardiorenal syndromes with vital energy deficiency, yang deficiency, and water accumulation as the main symptoms to tonify vital energy, warm yang, and promote urination. In this formula, *Cinnamomi ramulus* and *Atractylodis macrocephalae rhizoma* are used as minister medicines to help *Astragali radix* to tonify vital energy and warm yang. *Cinnamomi ramulus* is pungent, sweet and warm-natured, can warm yang and benefit vital energy, and *Atractylodis macrocephalae rhizoma* can invigorate spleen and dry dampness. Therefore, when *Atractylodis macrocephalae rhizoma* is combined with *Cinnamomi ramulus,* its warming effect is even greater, the spleen and vital energy are invigorated, and dampness is removed. *Paeoniae rubra radix* and *Spatholobi caulis* are both used as minister medicines to promote blood circulation and replenish blood, disperse stasis and alleviate pain in the treatment of the symptoms of blood stasis. *Poria* and *Alismatis rhizoma* are used as assistant medicines, and the combination of the two can multiply the effect of urination-promoting and dampness-draining. The compatibility combination of *Poria, Cinnamomi ramulus,* and *Atractylodis macrocephalae rhizoma* can promote yang and benefit vital energy, fortify spleen and promote urination, and it is warm but not dry, and is beneficial but not harsh. *Panacis majoris rhizoma* and *Ginkgo folium* are used as assistant medicines, which can promote blood circulation, remove stasis, and alleviate pain to help *Paeoniae rubra radix* and *Spatholobi caulis* to circulate the blood and remove stasis. In addition, *Panacis majoris rhizoma* can tonify the lung and nourish yin, and is combined with *Paeoniae rubra radix* to nourish yin and moisten the dryness. If blood is not flowing smoothly, it will induce water retention. If yang deficiency cannot warm and promote the circulation of body fluids, it will lead to the formation of phlegm and dampness. *Ginkgo folium* and *Alismatis rhizoma* also can resolve turbidity and lower the lipid to enhance the general effect of promoting blood circulation and promoting urination. *Citri fructus* and *Inulae flos* are both used as assistant medicines and can regulate vital energy and can resolve phlegm to regulate retention of phlegm and fluid caused by heart failure and cardiorenal syndromes. *Zingiberis rhizoma recens* and *Jujubae fructus* are both courier medicines. *Zingiberis rhizoma recens* is pungent and slightly warm, promotes movements without stagnation, and can help *Poria, Cinnamomi ramulus,* and *Atractylodis macrocephalae rhizoma* to warm middle burner and fortify the spleen. *Jujubae fructus* can not only tonify middle burner and benefit vital energy, but also coordinate the effects of other drugs. The combination of these drugs acts synergistically to warm yang, tonify vital energy, promote blood circulation, remove blood stasis, remove phlegm and promote urination. Therefore, the traditional Chinese medicine composition can significantly reduce the gene expression of natriuretic peptide B-type (*NPPB*), a gold index of heart failure induced by hypoxia/reoxygenation, reduce the expression of genes related to TGFβ-induced myocardial fibrosis and renal fibrosis, and significantly increase the blood flow rate and cardiac output in the zebrafish heart failure model. Clinical experience shows that the traditional Chinese medicine composition has good efficacy in improving the symptoms of patients with chronic heart failure and reducing the expression of NT-proBNP, indicating that the composition has a regulatory effect on the pathological developments such as myocardial ischemia, myocardial hypertrophy, myocardial fibrosis, renal fibrosis caused by heart failure and cardiorenal syndrome. It can effectively improve the cardiac function and renal function, and inhibit the progression of heart failure and inhibit cardiorenal fibrosis, thereby achieving the therapeutic effect of treating cardiorenal syndrome and heart failure.

### BRIEF DESCRIPTION OF DRAWINGS

FIG 1 shows the regulation effects of the traditional Chinese medicine composition on the expression regulation of natriuretic peptide B-type (*NPPB*).
FIG 2 shows the regulation effects of the traditional Chinese medicine composition on the expression of collagen type I alpha 1 (*COL1A1*) and collagen type V alpha 1 (*COL5A1*)*.*
FIG 3 shows the regulation effects of the traditional Chinese medicine composition on the expression of plasminogen activator inhibitor 1 (*SERPINE1*) and collagen type I alpha 1 (*COL1A1*)*.*
FIG 4 shows the regulation effects of the traditional Chinese medicine composition on the blood flow rate and cardiac output.

### DETAILED DESCRIPTION

The present disclosure provides a traditional Chinese medicine composition for use in the treatment of a cardiovascular and renal disease and a preparation method thereof. Those skilled in the art can refer to the contents of this disclosure and appropriately improve the process parameters to realize the present disclosure. It should be particularly pointed out that all similar replacements and modifications are apparent to those skilled in the art, and they are all considered to be included in the present disclosure. The method and the application of the present disclosure have been described through the preferred embodiments. It is obvious that those skilled in the art can change or appropriately modify and combine the method and application described herein without departing from the content, spirit and scope of the present disclosure to realize and apply the technology of the present disclosure.

In the present disclosure, the used raw materials and reagents are commercially available in the preparation of the formulation of the composition, the expression of *NPPB,* a gold index of heart failure induced by hypoxia/reoxygenation in AC16 cardiomyocytes, the influence on the expression of collagen type I alpha 1 (*COL1A1*) and collagen type V alpha 1 (*COL5A1*) genes in TGFβ-induced human cardiac fibroblasts, the influence on the expression of plasminogen activator inhibitor-1 (*SERPINE1*) and collagen type I alpha 1 (*COL1A1*), and the influence of the traditional Chinese medicine composition on the blood flow rate and cardiac output detected in the zebrafish heart failure model.

The present disclosure is further illustrated below in conjunction with examples.

### Example 1 Preparation of the traditional Chinese medicine composition

9 g of *Cinnamomi ramulus,* 10 g of *Atractylodis macrocephalae rhizoma,* 10 g of *Jujubae fructus,* 20 g of *Astragali radix,* 12 g of *Paeoniae rubra radix,* 10 g of *Spatholobi caulis,* 10 g of *Ginkgo folium,* 15 g of *Poria,* 9 g of *Zingiberis rhizoma recens,* 10 g of *Citri fructus,* 9 g of *Inulae flos,* 10 g of *Alismatis rhizoma,* 9 g of *Panacis majoris rhizoma,* and 6 g of *Nigellae semen* were separately weighted and crushed to obtain a powder with a fineness of 80 to 100 meshes. The powder was extracted with 15 times the volume of distilled water and standard reflux extraction was carried out for 2 h. The extracting solution was concentrated for 15 min to a volume of 2 mL. Under conditions of a vacuum degree of 5 Pa and a temperature of -55°C, the extract was freeze-dried by reducing the pressure and concentrating using a vacuum freeze dryer at a concentration ratio of 30 for 24 h to prepare the extract as a lyophilized powder. According to the proportion of the formula, the composition was prepared to obtain the traditional Chinese medicine composition, which was then dissolved in PBS to prepare a mother solution.

### Example 2 Effects of the traditional Chinese medicine composition on the expression of NPPB, a gold index of heart failure induced by hypoxia/reoxygenation in AC16 cardiomyocytes

1) AC16 cells were placed in a 12-well plate at 200,000 cells/well overnight. The cells in normal control group were cultured in a normal incubator (having an oxygen concentration of 20%) throughout the experiment. The cells in other groups were first placed in a tri-gas incubator and cultured in a sugar-free medium (having a glucose concentration of 0 g/L) under hypoxia (having an oxygen concentration of 1%) for 6 h, and then transferred into a normal incubator and cultured in a normal medium (having a glucose concentration of 3.15 g/L) for 24 h.
2) The cells were treated with 400 µg/mL of the traditional Chinese medicine composition prepared in Example 1 under the condition of hypoxia/reoxygenation for 30 h.
3) The cells were washed twice with PBS, and RNA was extracted and reverse transcribed to cDNA using a kit (Thermo, High-Capacity RNA-to-cDNA).
4) The qPCR reaction was carried out according to a procedure: 95°C for 3 min; followed by 40 cycles at 95°C for 3 s and 60°C for 30 s.
The primer sequences are shown below:
NPPB1:5'-TGGAAACGTCCGGGGTTACAG-3' (as shown in SEQ ID NO: 1)
NPPB2:5'-CTGATCCGGTCCATCTTCCT-3' (as shown in SEQ ID NO: 2)

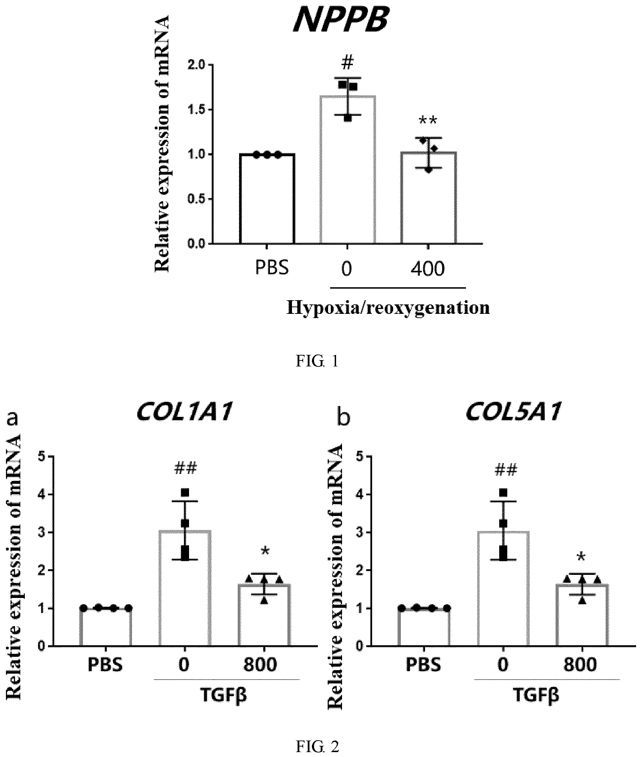

5) Data processing: The gene expression of the blank control group was taken as 1. In the drug-treated group, a gene expression greater than 1 indicated an up-regulation of gene expression, and less than 1 indicated down-regulation. As shown in FIG 1 and Table 1, the traditional Chinese medicine composition can inhibit regulation of the gene expression of B-type natriuretic peptide (*NPPB*) induced by hypoxia/reoxygenation in AC16 cell model, thus exerting the effect of anti-heart failure.

**Table 1**

| Measurement of *NPPB* | Experiment 1 | Experiment 2 | Experiment 3 | Mean |
|---|---|---|---|---|
| PBS | 1.00 | 1.00 | 1.00 | 1.00 |
| hypoxia/reoxygenation + 0 µg/mL of the traditional Chinese medicine composition | 1.76 | 1.78 | 1.41 | 1.65 |
| hypoxia/reoxygenation + 400 µg/mL of the traditional Chinese medicine composition | 1.07 | 1.16 | 0.83 | 1.02 |

### Example 3 Effects of the traditional Chinese medicine composition on the expression of collagen type I α1 (COL1A1) and collagen type V α1 (COL5A1) genes in TGFβ-induced human cardiac fibroblast cells

HCF cells were plated in a 12-well plate at 10,0000 cells per well overnight and were then treated with 10 ng/mL of TGFβ for 48 h.

2) A final concentration of 800 µg/mL of the traditional Chinese medicine composition prepared in Example 1 was added to treat the cells for 48 h. At the same time, a solvent control group was established (adding an equal volume of PBS to the cells).

3) The cells were washed twice with PBS, and RNA was extracted and reverse transcribed to cDNA using a kit (Thermo, High-Capacity RNA-to-cDNA).

4) The qPCR reaction was carried out according to a procedure: 95°C for 3 min; followed by 40 cycles at 95°C for 3 s and 60°C for 30 s.

The primer sequences are shown below:
COL1A1-5'-GAGGGCCAAGACGAAGACATC-3' (as shown in SEQ ID NO: 3)
COL1A1'-5'-CAGATCACGTCATCGCACAAC-3' (as shown in SEQ ID NO: 4)
COL5A1-5'-TACCCTGCGTCTGCATTTCC-3' (as shown in SEQ ID NO: 5)
COL5A1'-5'-GCTCGTTGTAGATGGAGACCA-3' (as shown in SEQ ID NO: 6)

As shown in FIG 2 and Table 2, the traditional Chinese medicine composition can inhibit the expression of collagen type I alpha 1 (*COL1A1*) and collagen type V alpha 1 (*COL5A1*) in TGFβ-induced human myocardial fibroblasts, thereby exerting the effect of anti-myocardial fibrosis.

**Table 2**

| Measurement of *COL1A1* | Experiment 1 | Experiment 2 | Experiment 3 | Experiment 4 | Mean |
|---|---|---|---|---|---|
| PBS | 1.00 | 1.02 | 1.00 | 1.00 | 1.01 |
| TGBβ+0 µg/mL of the traditional Chinese medicine composition | 2.35 | 4.05 | 3.24 | 2.55 | 3.05 |
| TGBβ+800 µg/mL of the traditional Chinese medicine composition | 1.77 | 1.77 | 1.79 | 1.22 | 1.64 |

| Measurement of *COL5A1* | Experiment 1 | Experiment 2 | Experiment 3 | Experiment 4 | Mean |
|---|---|---|---|---|---|
| PBS | 1.00 | 1.00 | 1.00 | 1.00 | 1.00 |
| TGBβ+0 µg/mL of the traditional Chinese medicine composition | 2.16 | 3.22 | 3.51 | 3.58 | 3.12 |
| TGBβ+800 µg/mL of the traditional Chinese medicine composition | 1.91 | 2.07 | 2.40 | 2.72 | 2.28 |

### Example 4 Effects of the traditional Chinese medicine composition on the expression of plasminogen activator inhibitor-1 (SERPINE1) and collagen type I alpha 1 (COL1A1) genes

1) HK2 cells were plated in a 12-well plate at 80,000 cells/well overnight and then subjected to serum-starvation for 24 h. Subsequently, 10 ng/mL of TGFβ was added to treat the cells for 48 h.
2) A final concentration of 800 µg/mL of the traditional Chinese medicine composition prepared in Example 1 was added to treat the cells for 48 h. At the same time, a solvent control group was established (adding an equal volume of PBS to the cells).
3) The cells were washed twice with PBS, and RNA was extracted and reverse transcribed to cDNA using a kit (Thermo, High-Capacity RNA-to-cDNA).
4) The qPCR reaction was carried out according to a procedure: 95°C for 3 min, followed by 40 cycles at 95°C for 3 s and 60°C for 30 s.
   The primer sequences are shown below:
   SERPINE1-5'-AGTGGACTTTTCAGAGGTGGA-3' (as shown in SEQ ID NO: 7)
   SERPINE1'-5'-GCCGTTGAAGTAGAGGGCATT-3' (as shown in SEQ ID NO: 8)
   COL1A1-5'-GAGGGCCAAGACGAAGACATC-3' (as shown in SEQ ID NO: 3)
   COL1A1'-5'-CAGATCACGTCATCGCACAAC-3' (as shown in SEQ ID NO: 4)
5) Data processing: The gene expression of the blank control group was taken as 1. In the drug-treated group, a gene expression greater than 1 indicated an up-regulation of gene expression, and less than 1 indicated down-regulation.

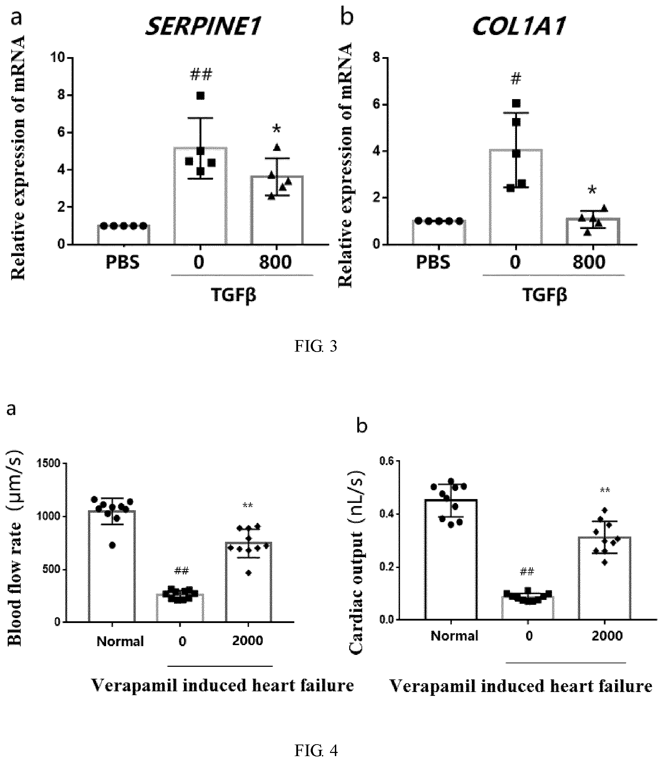

As shown in FIG 3 and Table 3, the traditional Chinese medicine composition can inhibit the expression of plasminogen activator inhibitor-1 of (*SERPINE1*) and collagen type I alpha 1 (*COL1A1*) in TGFβ-induced human renal tubular epithelial cells HK2, thereby exerting the effect of anti-renal fibrosis.

**Table 3**

| Measurement of *SERPINE1* | Experiment 1 | Experiment 2 | Experiment 3 | Experiment 4 | Experiment 5 | Mean |
|---|---|---|---|---|---|---|
| PBS | 1.00 | 1.00 | 1.00 | 1.00 | 1.00 | 1.00 |
| TGBβ+0 µg/mL of the traditional Chinese medicine composition | 4.46 | 5.02 | 7.98 | 3.93 | 4.39 | 5.16 |
| TGBβ+800 µg/mL of the traditional Chinese medicine composition | 2.62 | 3.41 | 5.24 | 3.10 | 3.76 | 3.63 |

| Measurement of *COL1A1* | Experiment 1 | Experiment 2 | Experiment 3 | Experiment 4 | Experiment 5 | Mean |
|---|---|---|---|---|---|---|
| PBS | 1.01 | 1.02 | 1.00 | 1.00 | 1.00 | 1.01 |
| TGBβ+0 µg/mL of the traditional Chinese medicine composition | 6.06 | 5.25 | 2.42 | 2.62 | 3.90 | 4.05 |
| TGBβ+800 µg/mL of the traditional Chinese medicine composition | 1.16 | 1.14 | 1.58 | 0.94 | 0.55 | 1.08 |

### Example 5 Effects of the traditional Chinese medicine composition on blood flow rate and cardiac output by using a zebrafish heart failure model

1) 2 dpf wild-type zebrafish of AB strain were randomly selected and placed in a 6-well plate at 30 zebrafish per well.
2) A final concentration of 2000 µg/mL of the traditional Chinese medicine composition prepared in Example 1 was added to treat the zebrafish for 4 h. At the same time, a solvent control group was established (adding an equal volume of sterile water to the sterile water).
3) Except for the normal control group (without verapamil hydrochloride treatment), the other groups were given verapamil hydrochloride in water and the zebrafish were treated at 28°C for 0.5 h to establish a zebrafish heart failure model.
4) 10 zebrafish were randomly selected from each experimental group and placed under the heartbeat blood flow analyzer to record a video. The data were collected using the ViewPoint Application Manager software. The blood flow rate and cardiac output of the zebrafish were analyzed.

As shown in FIG 4 and Table 4, the traditional Chinese medicine composition can increase the blood flow rate and cardiac output of the zebrafish heart failure model induced by verapamil, thereby exerting the effect of anti-heart failure.

### Example 6

Formula: 3 g of *Cinnamomi ramulus,* 25 g of *Atractylodis macrocephalae rhizoma,* 3 g of *Jujubae fructus,* 6 g of *Astragali radix,* 20 g of *Panacis majoris rhizoma,* 3 g of *Paeoniae rubra radix,* 6 g of *Spatholobi caulis,* 6 g of *Ginkgo folium,* 35 g of *Poria,* 20 g of *Zingiberis rhizoma recens,* 2 g of *Citri fructus,* 15 g of *Nigellae semen,* 20 g of *Inulae flos,* and 25 g of *Alismatis rhizoma.*

The preparation method was the same as that in Example 1.

### Example 7

Formula: 20 g of *Cinnamomi ramulus,* 3 g of *Atractylodis macrocephalae rhizoma,* 25 g of *Jujubae fructus,* 45 g of *Astragali radix,* 2 g of *Panacis majoris rhizoma,* 30 g of *Paeoniae rubra radix,* 25 g of *Spatholobi caulis,* 25 g of *Ginkgo folium,* 6 g of *Poria,* 3 g of *Zingiberis rhizoma recens,* 25 g of *Citri fructus,* 2 g of *Nigellae semen,* 2 g of *Inulae flos,* and 3 g of *Alismatis rhizoma.*

The preparation method was the same as that in Example 1.

**Table 4**

| Measur ement of blood flow rate | Normal | verapamil +0 µg/mL of the traditional Chinese medicine compositio n | verapamil +2000 µg/mL of the traditional Chinese medicine compositio n | Measure ment of cardiac output | Normal | verapamil +0 µg/mL of the traditional Chinese medicine compositio n | verapamil +2000 µg/mL of the traditional Chinese medicine compositio n |
|---|---|---|---|---|---|---|---|
| 1 | 1029.2 3 | 227.49 | 469.59 | 1 | 0.43 | 0.08 | 0.22 |
| 2 | 985.16 | 313.84 | 891.28 | 2 | 0.37 | 0.09 | 0.42 |
| 3 | 1114.5 5 | 305.77 | 681.82 | 3 | 0.52 | 0.10 | 0.29 |
| 4 | 1090.3 5 | 294.74 | 890.86 | 4 | 0.46 | 0.09 | 0.38 |
| 5 | 1162.0 9 | 211.75 | 694.95 | 5 | 0.38 | 0.07 | 0.36 |
| 6 | 1074.7 3 | 267.93 | 794.29 | 6 | 0.50 | 0.11 | 0.26 |
| 7 | 1069.5 4 | 214.17 | 707.49 | 7 | 0.50 | 0.10 | 0.33 |
| 8 | 1096.0 7 | 288.76 | 725.70 | 8 | 0.36 | 0.07 | 0.31 |
| 9 | 1142.1 0 | 232.31 | 703.00 | 9 | 0.48 | 0.08 | 0.26 |
| 10 | 731.23 | 274.76 | 909.23 | 10 | 0.51 | 0.09 | 0.30 |
| Mean | 1049.5 1 | 263.15 | 746.82 | Mean | 0.45 | 0.09 | 0.31 |

### Example 8 Preparation of aqueous decoction of a composition

In accordance with the proportion for each decoction piece of medicine, including 9 g of *Cinnamomi ramulus,* 10 g of *Atractylodis macrocephalae rhizoma,* 10 g of *Jujubae fructus,* 20 g of *Astragali radix,* 12 g of *Paeoniae rubra radix,* 10 g of *Spatholobi caulis,* 10 g of *Ginkgo folium,* 15 g of *Poria,* 9 g of *Zingiberis rhizoma recens,* 10 g of *Citri fructus,* 9 g of *Inulae flos,* 10 g of *Alismatis rhizoma,* 9 g of *Panacis majoris rhizoma,* and 6 g of *Nigellae semen,* 7 decoction pieces were weighed, added with 6 L of water, and decocted for 1 h to obtain a 7 day-supply of the aqueous decoction of the traditional Chinese medicine composition.

### Example 9 Effect of the aqueous decoction of the composition on symptoms and NT-proBNP of patients with chronic heart failure

Twelve patients were recruited. The patients had accepted basic western medicine treatment for heart failure (standardized basic western medicine therapy according the guidelines, with reference to 2022 AHA/ACC/HFSA Guideline for the Management of Heart Failure), including ARNI/ACEI/ARB, βeta-blockers, MRA, SGLT-2 inhibitors, cardiotonic agents, diuretics, antiplatelet agents, anticoagulants, statins, nitrates, antidiabetic agents, hypotensor, and the like, still had symptoms such as wheezing, shortness of breath, oedema and fatigue (see the statistics of the 'Before administration' part in the 'Improvement of symptoms' item shown in Table 5 for detail). The statistics of diagnosis on the basis of New York Heart Association (NYHA) Classification of Heart Failure are shown in Table 5. In the case of unchanged western medical treatment plan, these patients were further administered with the aqueous decoction of the traditional Chinese medicine combination prepared in Example 8 for 2 weeks or more. As shown in Table 5, these patients had a significant improvement in symptoms. The improvement was mainly as follows: in terms of wheezing of patients, a therapeutic effective rate was up to 100% (12/12); in terms of fatigue of patients, a therapeutic effective rate was up to 100% (12/12); in terms of oedema of patients (of which 2 patients had no oedema), a therapeutic effective rate was up to 90% (9/10); and in terms of shortness of breath of patients, a therapeutic effective rate was up to 66.7% (8/12). Among them, five patients were randomly detected for plasma NT-proBNP before administration of the aqueous decoction of the traditional Chinese medicine composition prepared in Example 8 and during or after the treatment course, and the levels of NT-proBNP decreased significantly (as shown in Table 6).

**Table 5**

| N u m b e r | S ex | A ge | Main diagnosis in Western medicine | Adminis tration Time (Day) | Improvement of symptoms (Traditional Chinese Medicine syndrome scores) | | | | | | | |
|---|---|---|---|---|---|---|---|---|---|---|---|---|
| | | | | | Wheezing | | Shortness of breath | | Oedema | | Fatigue | |
| | | | | | Bef ore adm inis trati on | After admin istrati on | Befor e admin istrati on | After admin istrati on | Befor e admin istrati on | After admin istrati on | Befor e admin istrati on | After admini stratio n |
| 1 | W m an | 67 | Coronary atheroscleroti c heart disease (NYHA class III) | 14 | 4 | 2↓ | 4 | 2↓ | 2 | 0↓ | 6 | 2↓↓ |
| 2 | w o m an | 76 | Coronary atheroscleroti c heart disease (NYHA class III) | 18 | 4 | 2↓ | 2 | 2 | 4 | 0↓↓ | 6 | 2↓↓ |
| 3 | M an | 63 | Dilated Cardiomyopa thy (NYHA class III) | 16 | 4 | 0↓↓ | 2 | 2 | 2 | 0↓ | 2 | 0↓ |
| 4 | M an | 82 | Coronary atheroscleroti c heart disease (NYHA class III) | 42 | 6 | 2↓↓ | 2 | 0↓ | 4 | 0↓↓ | 4 | 2↓ |
| 5 | M an | 39 | Coronary atheroscleroti c heart disease (NYHA class II) | 28 | 4 | 2↓ | 2 | 0↓ | 0 | 0 | 2 | 0↓ |
| 6 | M an | 54 | Acute exacerbation of chronic cardiac insufficiency (NYHA class III) | 14 | 4 | 2↓ | 4 | 2↓ | 2 | 0↓ | 2 | 0↓ |
| 7 | M an | 79 | Acute exacerbation of chronic cardiac insufficiency (NYHA class II) | 14 | 4 | 2↓ | 2 | 0↓ | 4 | 0↓↓ | 4 | 2↓ |
| 8 | W m an | 70 | Coronary atheroscleroti c heart disease (NYHA class III) | 14 | 4 | 2↓ | 2 | 2 | 4 | 0↓↓ | 2 | 0↓ |
| 9 | M an | 69 | Hypertrophic cardiomyopat hy, heart failure, (NYHA class IV) | 14 | 6 | 4↓ | 4 | 4 | 4 | 2↓ | 4 | 2↓ |
| 1 0 | M an | 51 | Dilated cardiomyopat hy, (NYHA class III) | 14 | 6 | 4↓ | 4 | 2↓ | 2 | 2 | 6 | 2↓↓ |
| 1 1 | M an | 78 | Chronic heart failure, dilated cardiomyopat hy, (NYHA class III) | 17 | 4 | 2↓ | 4 | 2↓ | 0 | 0 | 2 | 0↓ |
| 1 2 | M an | 41 | Acute exacerbation of chronic cardiac insufficiency (NYHA class III) | 17 | 4 | 0↓↓ | 2 | 0↓ | 2 | 0↓ | 2 | 0↓ |

In Table 5, the expression 'Before administration' means that scores were collected after taking the western medicine in the base treatment of heart failure in Example 9, and the expression 'After administration' means that scores were collected after taking the western medicine in the base treatment of heart failure in Example 9 and taking the aqueous decoction of the traditional Chinese medicine composition prepared in Example 8.

**Table 6**

| Number | | 1 | 2 | 6 | 11 | 12 |
|---|---|---|---|---|---|---|
| Sex | | Woman | Woman | Man | Man | Man |
| Age | | 67 | 76 | 54 | 78 | 41 |
| NT-pr oBNP level (pg/m L) | Before administr ation | 1262 | >30000 | 7285 | 4583 | 20827 |
| | After administr ation | 1012 (11 days of administrati on) | 21421 (18 days of administrati on) | 4682 (4 days of administrati on) | 3327 (3 days of administra tion) | 2389 (11 days of administrat ion) |
| | Change Rate | -19.8%↓ | -28.6%↓ | -35.7%↓ | -27.4 %↓ | -88.5%↓ |

In Table 6, the expression 'Before administration' means that data was collected after taking the western medicine in the base treatment of heart failure in Example 9, and the expression 'After administration' means that data was collected after taking the western medicine in the base treatment of heart failure in Example 9 and taking the aqueous decoction of the traditional Chinese medicine composition prepared in Example 8.

### Example 10 Effects of the aqueous decoction of the composition on liver and kidney functions of patients with chronic heart failure

Twelve patients were recruited in Example 9. The patients were administered with the aqueous decoction of the traditional Chinese medicine composition prepared in Example 8 for 2 weeks or more. As shown in Table 7, all the following items related to liver and kidney functions including alanine transaminase detected by using a kit for alanine transaminase assay (Beckman Coulter Inc), aspartate aminotransferase detected by using a kit for aspartate aminotransferase assay (Beckman Coulter Inc), blood urea nitrogen detected by using a kit for blood urea nitrogen assay (Beckman Coulter Inc), and serum creatinine detected by using a kit for serum creatinine assay (Beckman Coulter Inc) exhibited no significant difference in measured value.

**Table 7**

| Item | Before administration (x±s) | After administration (x±s) | p value |
|---|---|---|---|
| Alanine transaminase (ALT, U/L) | 17.89±5.34 | 20.58±9.08 | 0.482 |
| Aspartate aminotransferase (U/L) | 23.43±6.23 | 25.81±10.38 | 0.586 |
| Blood urea nitrogen (mmol/L) | 8.35±2.62 | 8.17±2.81 | 0.899 |
| Serum creatinine (µmol/L) | 89.25±27.54 | 88.5±26.84 | 0.957 |

The traditional Chinese medicine composition for use in the treatment of a cardiovascular and renal disease and preparation method thereof provided by the present disclosure are described in detail above. The principle and implementation of the present disclosure are illustrated by using specific embodiments herein. The above descriptions of the embodiments are only used to facilitate understanding of the method and the core idea of the present disclosure. It should be noted that, those skilled in the art can make several improvements and modifications to the present disclosure without departing from the principle of the present disclosure, and the improvements and modifications shall fall within the protection scope of the claims.

### SEQUENCE LISTING

<?xml version="1.0" encoding="UTF-8"?>
<!DOCTYPE ST26SequenceListing SYSTEM "ST26SequenceListing_V1_3.dtd" PUBLIC
"-//WIPO//DTD Sequence Listing 1.3//EN">
<ST26SequenceListing productionDate="2023-05-30" softwareVersion="2.1.1"
softwareName="WIPO Sequence" fileName="OP230764.xml"
dtdVersion="V1_3"><ApplicantFileReference>OP230764</ApplicantFileReferenc
e><EarliestPriorityApplicationIdentification><IPOfficeCode>CN</IPOfficeCo
de><ApplicationNumberText>202210615447.4</ApplicationNumberText><FilingDa
te>2022-06-01</FilingDate></EarliestPriorityApplicationIdentification><Ap
plicantName languageCode="zh">BEIJING CAPITALBIO PHARMA CO.,
LTD.</ApplicantName><ApplicantNameLatin>Beijing Capitalbio Technology
Co.Ltd</ApplicantNameLatin><InventionTitle languageCode="zh"> TRADITIONAL
CHINESE MEDICINE COMPOSITION FOR TREATING CARDIOVASCULAR AND KIDNEY DISEASES
AND METHOD FOR PREPARING SAME
</InventionTitle><SequenceTotalQuantity>8</SequenceTotalQuantity><Sequenc
eData
sequenceIDNumber="1"><INSDSeq><INSDSeq_length>20</INSDSeq_length><INSDSeq
_moltype>DNA</INSDSeq_moltype><INSDSeq_division>PAT</INSDSeq_division><IN
SDSeq_feature-table><INSDFeature><INSDFeature_key>source</INSDFeature_key
><INSDFeature_location>1..20</INSDFeature_location><INSDFeature_quals><IN
SDQualifier><INSDQualifier_name>mol_type</INSDQualifier_name><INSDQualifi
er_value>other DNA</INSDQualifier_value></INSDQualifier><INSDQualifier
id="q2"><INSDQualifier_name>organism</INSDQualifier_name><INSDQualifier_v
alue>synthetic
construct</INSDQualifier_value></INSDQualifier></INSDFeature_quals></INSD
Feature></INSDSeq_feature-table><INSDSeq_sequence>tggaaacgtccgggttacag</I
NSDSeq_sequence></INSDSeq></SequenceData><SequenceData
sequenceIDNumber="2"><INSDSeq><INSDSeq_length>20</INSDSeq_length><INSDSeq
_moltype>DNA</INSDSeq_moltype><INSDSeq_division>PAT</INSDSeq_division><IN
SDSeq_feature-table><INSDFeature><INSDFeature_key>source</INSDFeature_key
><INSDFeature_location>1..20</INSDFeature_location><INSDFeature_quals><IN
SDQualifier><INSDQualifier_name>mol_type</INSDQualifier_name><INSDQualifi
er_value>other DNA</INSDQualifier_value></INSDQualifier><INSDQualifier
id="q3"><INSDQualifier_name>organism</INSDQualifier_name><INSDQualifier_v
alue>synthetic
construct</INSDQualifier_value></INSDQualifier></INSDFeature_quals></INSD
Feature></INSDSeq_feature-table><INSDSeq_sequence>ctgatccggtccatcttcct</I
NSDSeq_sequence></INSDSeq></SequenceData><SequenceData
sequenceIDNumber="3"><INSDSeq><INSDSeq_length>21</INSDSeq_length><INSDSeq
_moltype>DNA</INSDSeq_moltype><INSDSeq_division>PAT</INSDSeq_division><IN
SDSeq_feature-table><INSDFeature><INSDFeature_key>source</INSDFeature_key

><INSDFeature_location>1..21</INSDFeature_location><INSDFeature_quals><IN
SDQualifier><INSDQualifier_name>mol_type</INSDQualifier_name><INSDQualifi
er_value>other DNA</INSDQualifier_value></INSDQualifier><INSDQualifier
id="q4"><INSDQualifier_name>organism</INSDQualifier_name><INSDQualifier_v
alue>synthetic
construct</INSDQualifier_value></INSDQualifier></INSDFeature_quals></INSD
Feature></INSDSeq_feature-table><INSDSeq_sequence>gagggccaagacgaagacatc</
INSDSeq_sequence></INSDSeq></SequenceData><SequenceData
sequenceIDNumber="4"><INSDSeq><INSDSeq_length>21</INSDSeq_length><INSDSeq
_moltype>DNA</INSDSeq_moltype><INSDSeq_division>PAT</INSDSeq_division><IN
SDSeq_feature-table><INSDFeature><INSDFeature_key>source</INSDFeature_key
><INSDFeature_location>1..21</INSDFeature_location><INSDFeature_quals><IN
SDQualifier><INSDQualifier_name>mol_type</INSDQualifier_name><INSDQualifi
er_value>other DNA</INSDQualifier_value></INSDQualifier><INSDQualifier
id="q5"><INSDQualifier_name>organism</INSDQualifier_name><INSDQualifier_v
alue>synthetic
construct</INSDQualifier_value></INSDQualifier></INSDFeature_quals></INSD
Feature></INSDSeq_feature-table><INSDSeq_sequence>cagatcacgtcatcgcacaac</
INSDSeq_sequence></INSDSeq></SequenceData><SequenceData
sequenceIDNumber="5"><INSDSeq><INSDSeq_length>20</INSDSeq_length><INSDSeq
_moltype>DNA</INSDSeq_moltype><INSDSeq_division>PAT</INSDSeq_division><IN
SDSeq_feature-table><INSDFeature><INSDFeature_key>source</INSDFeature_key
><INSDFeature_location>1..20</INSDFeature_location><INSDFeature_quals><IN
SDQualifier><INSDQualifier_name>mol_type</INSDQualifier_name><INSDQualifi
er_value>other DNA</INSDQualifier_value></INSDQualifier><INSDQualifier
id="q6"><INSDQualifier_name>organism</INSDQualifier_name><INSDQualifier_v
alue>synthetic
construct</INSDQualifier_value></INSDQualifier></INSDFeature_quals></INSD
Feature></INSDSeq_feature-table><INSDSeq_sequence>taccctgcgtctgcatttcc</I
NSDSeq_sequence></INSDSeq></SequenceData><SequenceData
sequenceIDNumber="6"><INSDSeq><INSDSeq_length>21</INSDSeq_length><INSDSeq
_moltype>DNA</INSDSeq_moltype><INSDSeq_division>PAT</INSDSeq_division><IN
SDSeq_feature-table><INSDFeature><INSDFeature_key>source</INSDFeature_key
><INSDFeature_location>1..21</INSDFeature_location><INSDFeature_quals><IN
SDQualifier><INSDQualifier_name>mol_type</INSDQualifier_name><INSDQualifi
er_value>other DNA</INSDQualifier_value></INSDQualifier><INSDQualifier
id="q7"><INSDQualifier_name>organism</INSDQualifier_name><INSDQualifier_v
alue>synthetic
construct</INSDQualifier_value></INSDQualifier></INSDFeature_quals></INSD
Feature></INSDSeq_feature-table><INSDSeq_sequence>gctcgttgtagatggagacca</
INSDSeq_sequence></INSDSeq></SequenceData><SequenceData
sequenceIDNumber="7"><INSDSeq><INSDSeq_length>21</INSDSeq_length><INSDSeq
_moltype>DNA</INSDSeq_moltype><INSDSeq_division>PAT</INSDSeq_division><IN

SDSeq_feature-table><INSDFeature><INSDFeature_key>source</INSDFeature_key
><INSDFeature_location>1..21</INSDFeature_location><INSDFeature_quals><IN
SDQualifier><INSDQualifier_name>mol_type</INSDQualifier_name><INSDQualifi
er_value>other DNA</INSDQualifier_value></INSDQualifier><INSDQualifier
id="q8"><INSDQualifier_name>organism</INSDQualifier_name><INSDQualifier_v
alue>synthetic
construct</INSDQualifier_value></INSDQualifier></INSDFeature_quals></INSD
Feature></INSDSeq_feature-table><INSDSeq_sequence>agtggacttttcagaggtgga</
INSDSeq_sequence></INSDSeq></SequenceData><SequenceData
sequenceIDNumber="8"><INSDSeq><INSDSeq_length>21</INSDSeq_length><INSDSeq
_moltype>DNA</INSDSeq_moltype><INSDSeq_division>PAT</INSDSeq_division><IN
SDSeq_feature-table><INSDFeature><INSDFeature_key>source</INSDFeature_key
><INSDFeature_location>1..21</INSDFeature_location><INSDFeature_quals><IN
SDQualifier><INSDQualifier_name>mol_type</INSDQualifier_name><INSDQualifi
er_value>other DNA</INSDQualifier_value></INSDQualifier><INSDQualifier
id="q9"><INSDQualifier_name>organism</INSDQualifier_name><INSDQualifier_v
alue>synthetic
construct</INSDQualifier_value></INSDQualifier></INSDFeature_quals></INSD
Feature></INSDSeq_feature-table><INSDSeq_sequence>gccgttgaagtagagggcatt</
INSDSeq_sequence></INSDSeq></SequenceData></ST26SequenceListing>

## Claims

1. A traditional Chinese medicine composition, comprising the following components or processed products thereof in parts by weight: 3 to 20 parts of *Cinnamomi ramulus,* 3 to 25 parts of *Atractylodis macrocephalae rhizoma,* 3 to 25 parts of *Jujubae fructus,* 6 to 45 parts of *Astragali radix,* 2 to 20 parts of *Panacis majoris rhizoma,* 3 to 30 parts of *Paeoniae rubra radix,* 6 to 25 parts of *Spatholobi caulis,* 6 to 25 parts of *Ginkgo folium,* 6 to 35 parts of *Poria,* 3 to 20 parts of *Zingiberis rhizoma recens,* 2 to 25 parts of *Citri fructus,* 2 to 15 parts of *Nigellae semen,* 2 to 20 parts of *Inulae flos,* and 3 to 25 parts of *Alismatis rhizoma.*

2. The traditional Chinese medicine composition according to claim 1, comprising the following components or processed products thereof: 9 parts of *Cinnamomi ramulus,* 10 parts of *Atractylodis macrocephalae rhizoma,* 10 parts of *Jujubae fructus,* 20 parts of *Astragali radix,* 9 parts of *Panacis majoris rhizoma,* 12 parts of *Paeoniae rubra radix,* 10 parts of *Spatholobi caulis,* 10 parts of *Ginkgo folium,* 15 parts of *Poria,* 9 parts of *Zingiberis rhizoma recens,* 10 parts of *Citri fructus,* 6 parts of *Nigellae semen,* 9 parts of *Inulae flos,* and 10 parts of *Alismatis rhizoma.*

3. The traditional Chinese medicine composition according to claim 1 or 2, wherein the *Panacis majoris rhizoma* is replaced by Ginseng radix et rhizoma, Ginseng radix et Rhizoma rubra, Codonopsis radix, Panacis japonici rhizoma, Panacis quinquefolii radix, and/ or Pseudostellariae radix, and/or
the *Atractylodis macrocephalae rhizoma* is stir-baked *Atractylodis macrocephalae rhizoma* or stir-baked *Atractylodis macrocephalae rhizoma* with bran, and/or
the *Astragali radix* is Radix astragali preparata, and/or
the *Paeoniae rubra radix* is replaced by Paeoniae radix alba, and/or
the *Zingiberis rhizoma recens* is dried *Zingiberis rhizoma recens* or pickled *Zingiberis rhizoma recens.*

4. A method for preparing the traditional Chinese medicine composition according to any one of claims 1 to 3, comprising extracting the components or processed products thereof of the traditional Chinese medicine composition in a formulated amount with water.

5. The method according to claim 4, wherein the method further comprises steps of concentrating and/or drying after the extracting with water.

6. The method according to claim 4 or 5, wherein the method further comprises crushing the components or processed products thereof of the traditional Chinese medicine composition in a formulated amount to obtain a fine powder, and then extracting with water, concentrating, and freeze-drying.

7. The method according to any one of claims 4 to 6, wherein the extracting with water comprises decocting with water.

8. Use of the traditional Chinese medicine composition according to any one of claims 1 to 3 and/or the traditional Chinese medicine composition prepared by the method according to any one of claims 4 to 7 in the manufacture of a medicament for the regulation of cardiac and renal functions.

9. Use of the traditional Chinese medicine composition according to any one of claims 1 to 3 and/or the traditional Chinese medicine composition prepared by the method according to any one of claims 4 to 7 in the manufacture of a medicament for the prevention and/or treatment heart failure, cardiorenal syndrome and/or coronary heart disease.

10. Use of the traditional Chinese medicine composition according to any one of claims 1 to 3 and/or the traditional Chinese medicine composition prepared by the method according to any one of claims 4 to 7 in the manufacture of a medicament for increasing the blood flow rate and/or cardiac output in a zebrafish model of heart failure induced by verapamil.

11. Use of the traditional Chinese medicine composition according to any one of claims 1 to 3 and/or the traditional Chinese medicine composition prepared by the method according to any one of claims 4 to 7 in the manufacture of a medicament for the inhibition of myocardial ischaemia, myocardial hypertrophy, myocardial fibrosis and/or renal fibrosis.

12. Use of the traditional Chinese medicine composition according to any one of claims 1 to 3 and/or the traditional Chinese medicine composition prepared by the method according to any one of claims 4 to 7 in the manufacture of a medicament for the improvement of a symptom of a patient with chronic heart failure and/or reduction of the NT-proBNP expression.

13. Use of the traditional Chinese medicine composition according to any one of claims 1 to 3 and/or the traditional Chinese medicine composition prepared by the method according to any one of claims 4 to 7 in the manufacture of a medicament for the inhibition of the expression of *NPPB* gene, *COL1A1* gene, *COL5A1* gene and/or *SERPINE1* gene.

14. A medicament, comprising the traditional Chinese medicine composition according to any one of claims 1 to 3 and/or the traditional Chinese medicine composition prepared by the method according to any one of claims 4 to 7, and a pharmaceutically acceptable excipient.

15. Use of the traditional Chinese medicine composition according to any one of claims 1 to 3, the traditional Chinese medicine composition prepared by the method according to any one of claims 4 to 7, or the medicament according to claim 14 in the treatment of a disease selected from the group consisting of:
(I) heart failure,
(II) cardiorenal syndrome,
(III) coronary heart disease,
(IV) myocardial ischaemia,
(V) myocardial hypertrophy,
(VI) myocardial fibrosis, and
(VII) renal fibrosis.

16. A method for treating a disease, comprising administering the traditional Chinese medicine composition according to any one of claims 1 to 3, the traditional Chinese medicine composition prepared by the method according to any one of claims 4 to 7, and/or the medicament according to claim 14, wherein the disease is selected from the group consisting of:
(I) heart failure,
(II) cardiorenal syndrome,
(III) coronary heart disease,
(IV) myocardial ischaemia,
(V) myocardial hypertrophy,
(VI) myocardial fibrosis, and
(VII) renal fibrosis.
